# EUROPEAN PATENT SPECIFICATION

(11) **EP 2 800 507 B1**
(45) Date of publication and mention of the grant of the patent: **23.08.2023**
(21) Application number: 13705524.0
(22) Date of filing: 07.01.2013
(51) Int. Cl.: A61B 5/00, A61B 3/113, A61B 5/16

(54) **APPARATUS FOR PSYCHIATRIC EVALUATION**
VORRICHTUNG FÜR EINE PSYCHIATRISCHE AUSWERTUNG
APPAREIL D'ÉVALUATION PSYCHIATRIQUE

(30) Priority: 05.01.2012 GB 201200122
(43) Date of publication of application: 12.11.2014
(73) Proprietor: University Court Of The University Of Aberdeen, Aberdeen AB24 3FX (GB)
(72) Inventor: BENSON, Philip, Aberdeen, Aberdeenshire AB39 3QJ (GB); ST.CLAIR, David, Edinburgh, EH3 6QZ (GB); BEEDIE, Sara, Aberdeen, Aberdeenshire AB15 8HS (GB)
(74) Representative: Ablett, Graham Keith
(86) International application number: PCT/GB2013/050016
(87) International publication number: WO 2013/102768

(56) References cited:
- BESTELMEYER P E G ET AL: "Global visual scanning abnormalities in schizophrenia and bipolar disorder", SCHIZOPHRENIA RESEARCH, ELSEVIER, NETHERLANDS, vol. 87, no. 1-3, 1 October 2006 (2006-10-01), pages 212-222, XP027976460, ISSN: 0920-9964 [retrieved on 2006-10-01]
- BENSON P J ET AL: "MULTIVARIATE EYE MOVEMENT PSYCHOPHYSIOLOGY ACCURATELY DIFFERENTIATES SCHIZOPHRENIA CASES FROM UNAFFECTED CONTROLS", SCHIZOPHRENIA RESEARCH, ELSEVIER, NETHERLANDS, vol. 117, no. 2-3, 1 April 2010 (2010-04-01), pages 248-249, XP026979778, ISSN: 0920-9964, DOI: 10.1016/J.SCHRES.2010.02.383 [retrieved on 2010-03-20]
- Sarah A Beedie: "ATYPICAL VIEWING BEHAVIOUR IN SCHIZOPHRENIA", EThOS , 1 January 2009 (2009-01-01), XP055059304, Retrieved from the Internet: URL:http://ethos.bl.uk/OrderDetails.do?did =1&uin=uk.bl.ethos.499304 [retrieved on 2013-04-11]
- BEEDIE: "Smooth pursuit and visual scanpaths: Independence of two candidate oculomotor risk markers for schizophrenia", WORLD JOURNAL OF BIOLOGICAL PSYCHIATRY, INFORMA HEALTHCARE, UK, vol. 13, 1 January 2012 (2012-01-01), pages 200-210, XP008161394, ISSN: 1562-2975, DOI: 10.3109/15622975.2011.566628

## Description

This invention relates to an apparatus for use in psychiatric evaluation, more particularly to such a psychiatric evaluation based on analysing eye movements of a patient.

In this regard, many efforts have been made to date to identify traits, bio-markers, behaviour, and symptoms associated with particular psychiatric illnesses in order to assist in distinguishing affected individuals.

For example, a discovery of abnormal eye movements present in a proportion of unmedicated psychotic patients was made over 100 years ago. However, in spite of this early discovery, efforts to identify stable trait markers associated with illnesses such as schizophrenia that can accurately delineate sufferers from normal control test participants (control subjects) have met with limited success.

Furthermore, only very limited analysis of the multiple and subtle variations embedded in the many components that define eye tracking performance in schizophrenia have been performed, despite the apparent robustness of the many individual deficits in eye movement performance reported in schizophrenics and their relatives.

Whilst scientists are achieving a better understanding of the human mind and have more advance technologies at their disposal for analysis, it remains difficult to readily and accurately identify and diagnose current or potential affected individuals.

In this connection, psychiatric diagnoses are in practice currently symptom-based with only face validity, essentially without biomakers that can externally validate a diagnosis of mental illness. This is especially problematic where individuals and patients do not display the core features by which the most likely diagnosis is defined or they display features that are shared with more than one diagnostic category. Furthermore, in such cases, the diagnosis may not become apparent until the individual has been expertly assessed by careful observations over an extended period of time. Genotypic information may in the future help with some of these uncertainties but at present they are no better than clinical evaluation at predicting risk of specific disorders. Clinical evaluations require a significant amount of time, expertise and hence cost. There also remains the uncertainty as to whether an illness can in any event be diagnosed without the full diagnostic criteria being met.

In this connection, studies to date into eye movement abnormalities have not been able to define features that in psychiatric patients are either sensitive enough or specific enough to be of clinical value. Although there are group differences there is too much overlap between affected cases and control subjects and between the different diagnostic categories within the affected cases. For example, there is considerable evidence that individuals with schizophrenia and their biological family members perform poorly on Smooth Pursuit Eye Movements (SPEM) tests. However, when the sensitivity is set high, even a proportion of control subjects perform poorly, ie there is an inverse correlation between sensitivity and specificity.

A further example of a general trait of individuals with schizophrenia is in relation to saccades. In general, anti-saccades tests have been used to try to identify schizophrenia cases: however, such tests are well known to be very difficult to perform by both cases and control subjects. These tests have consequently been consistently error-prone with considerable variation in the results/error rates between studies and individuals. It is known that such tests have also been significantly affected by the age of the participants.

Furthermore, individuals with schizophrenia are also known to have poor fixation control. They have trouble with gaze stability and glissades. However, currently available tests are not able to produce consistent confirmatory results.

In relation to above, many studies have also found that schizophrenia's eye movements are frequently confined to a limited area of the stimulus or are concentrated on selected features, and scanning is atypical in contextual perception, recognition, search and free-viewing tasks.

Relatives and siblings have also showed the trait of restricted atypical scanning. However, the abnormal fixation combined with scanning tasks in schizophrenia have not been studied and no substantial findings have emerged. In consequence eye movement tests such as SPEM, anti-saccades, fixation/gaze maintenance and exploratory scanpaths are historically known to only partially discriminate schizophrenia cases from control subjects. Such poor discrimination between psychiatric patients and control subjects means that these tests have not been viewed as clinically/diagnostically useful since the rate of mis-diagnosis was unacceptably high.

BESTELMEYER ET AL: "Global visual scanning abnormalities in schizophrenia and bipolar disorder", SCHIZOPHRENIA RESEARCH, ELSEVIER, NETHERLANDS, vol. 87, no. 1-3, 1 October 2006, 5 pages 212-222, discloses a study designed to determine whether abnormal scanning of images in schizophrenic patients is specific to images with social content or extends to other types of stimuli. In the study, patients were shown a series or images with or without socially important content while10 their eye movements were recorded.

BENSON ET AL: "Multi variate eye movement psychophysiology accurately differentiates schizophrenia cases from unaffected controls", SCHIZOPHRENIA RESEARCH, ELSEVIER, NETHERLANDS, 15 vol. 117, no. 2-3, 1 April 2010, pages 248-249, discloses a study in which eye tracking performance of schizophrenic patients was measured using smooth pursuit, fixation stability and scanpath formation. Statistical models were then used to evaluate composite fixation and saccade activity 20 in each participant group.

SARAH A BEEDIE: "Atypical viewing behaviour in schizophrenia", EThOS, 1 January 2009, discloses a study involving the combination of at least a free-viewing test and 25 a smooth pursuit test to improve the discrimination between healthy and schizophrenic subjects.

BEEDIE: "Smooth pursuit and visual scanpaths: Independence of two candidate oculomotor risk markers for schizophrenia", WORLD JOURNAL OF BIOLOGICAL PSYCHIATRY, INFORMA HEALTHCARE, UK vol.13, 1 January 2021, pages 200-210, discloses a study of eye tracking disfunction and scanpath disturbance in individuals with schizophrenia.

Accordingly it is an object of the present invention to overcome the above problems associated with such known apparatus and methods.

According to the present invention, there is provided an apparatus according to claim 1.

In this connection, the present invention is concerned with developing apparatus which can be used to help to diagnose and classify individuals with psychiatric symptoms using criteria that are not based on face validity but rather have validity which is external to the symptoms themselves. This newly developed apparatus can as such exploit a statistical combination of performance measures from a collection of interactive tests to classify individuals.

In this connection, the apparatus uses the fundamentals of a free-viewing test to ensure that the influence of factors other than physiological conditions of the test participants can be minimised so as to reduce noisy data within the results. Under a controlled test environment, the patient is simply asked to look at the succession of images being displayed so that while viewing takes place the eye movements can be tracked. This aspect differs from other known tests in that the patients are not required to perform particular tasks which could introduce unnecessary noise into the data. The applicants have found that adaptation of the free view test in this particular application affords highly effective results for use in evaluating a psychological profile of a patient. The images shown to the patient are specifically chosen to be of neutral emotion, namely images whose content is intended to have a low risk of evoking raised emotions. The images have a moderate, undisturbing tranquil quality. Such images have proven to be particularly effective in the context of the present invention in producing extremely highly performing data.

Conveniently, the control means provides that a total of 42 images are displayed. The total number of images used in the test may change depending on the need of the tests, however, once the total number is set then it should be maintained for each of the free-viewing tests performed. This will maintain validity of the results data for evaluation. Nevertheless, the total of 42 images have been proved to produce a satisfactory result in relation to providing schemata for schizophrenia biomarkers. Conveniently, the images are presented in a predetermined sequence.

Conveniently, the apparatus further comprises a memory means for storing a plurality of neutral images, and said control means provides that each of said images from said memory means are displayed in turn in a predetermined sequence.

Preferably, the control means provides that the images are displayed for a period of 5 to 10 seconds. In general individuals with schizophrenia take longer than unaffected individuals to begin engaging in picture viewing. Therefore, shorter durations may not yield sufficient eye movements and alternatively, longer durations might adversely affect engagement with the images and will certainly increase the duration of the test causing unnecessary fatigue.

The control means is configured to control the apparatus to additionally perform smooth pursuit and fixation tests. Each of these tests are modified and enhanced to simplify the test so that virtually all patients and control subjects can undertake these successfully. These simplified tests focus and capitalise on overlooked and misused earlier discovery of abnormal eye movements in association with a particular mental disease/disorder, excluding any unnecessary reliance on cognitive behaviours of the test participants. Therefore the apparatus enables an alternative method of measuring eye movements to the currently used approaches in a much more cost-effective way to diagnose participants.

Conveniently, the control means takes results data from said smooth pursuit and fixation tests, and uses them to refine said first results data. In this regard, after collecting results data from different tests and using them in the analyses, the integrity of the data will be enhanced as it is cross validated across the tests for determining the possible traits. The distinctions between the sufferers and the control subjects are as a result shown more readily.

Preferably, the control means uses an algorithm to attribute relative importance to the results of the first results data set, and result data sets from said smooth pursuit and fixation tests. As such, the respective results are given a relative importance in the overall psychiatric evaluation. Training efficacy can be monitored using cross-validation to ensure a fair description of accuracy is obtained.

In preferred embodiments, the algorithm uses measures from 3 tests, namely free (picture) viewing, Lissajous 0.2Hz pursuit and fixation stability. Conveniently, the algorithm uses 10 such measures.

For a model based on a group comprising control + schizophrenia + bipolar + major depressive disorder, such measures are 1) Free Viewing (fixation dispersion), 2) Pursuit-Lissajous 0.2Hz (gain horizontal), 3) Pursuit-Lissajous 0.2Hz (gain vertical), 4) Pursuit-Horizontal 0.4 Hz (saccade duration), 5) Pursuit-Lissajous 0.4Hz (saccade frequency), 6) Free viewing (saccade average velocity), 7) Fixation-Distracter (scanpath length), 8) Free Viewing (saccade peak velocity), 9) Pursuit-Horizontal 0.4Hz (saccade peak velocity), and 10) Pursuit-Horizontal 0.4Hz (gain horizontal).

For such a model, the importance attributed to each measure out of 100 is given as: 1) 100, 2) 66.9, 3) 58.0, 4) 41.9, 5) 33.2, 6) 31.4, 7) 31.3, 8) 27.7, 9) 27.6 and 10) 25.8. In this respect, the relative importance of the Free Viewing (fixation dispersion) measure is apparent.

In preferred embodiments, the importance weighting of the Free-viewing (fixation dispersion) measure is from 85 to 100 out of 100.

The apparatus of the present invention may be used to perform a method for psychiatric evaluation comprising: displaying a succession of different neutral images to a patient whilst simultaneously tracking eye movement of the patient; recording a patient's eye movement as eye movement data in response to the display of said succession of images; wherein said images are displayed to the patient in a predetermined order and for a predetermined period of time; and collecting said eye movement data to produce a first results data set for use in combination with a second results data set obtainable from performing a smooth pursuit test and a fixation test, in identifying a psychiatric profile of the patient.

This is in essence a method based around a free viewing test. The images are neutral or bland, namely they are intentionally chosen so as not to provoke a strong emotional response. They have a moderate, undisturbing tranquil quality. This has proven to be highly effective in producing extremely highly performing data.

Preferably, a total of 42 images are displayed. Conveniently, said images are stored and selected in turn in a predetermined sequence and the images are displayed for a period of 5 to 10 seconds, preferably 8 seconds.

The control means is configured to control the apparatus to carrying out a smooth pursuit test and a fixation test. Results data are taken from said tests, and used to refine said first results data.

The control means preferably uses an algorithm to weight the first results data set, and the result data set from said one or more smooth pursuits test, fixation test and saccade test.

An importance weighting is preferably attributed to measures of the first results data set, and second result data set from said smooth pursuit and fixation tests.

Conveniently, 10 measures are used for building an evaluation model.

The measures preferably include a free viewing (fixation dispersion) measure.

Conveniently, said free viewing (fixation dispersion) measure is given an importance weighting of 85-100 out of 100.

The results data sets are preferably refined using preselected statistical methods.

The apparatus of the present invention may be configured for use in a method which may further comprise: repeating the method to obtain a number of sets of data from different patient groups; building a data bank for modelling purposes using at least two different statistical modelling techniques; and cross-validating the models. In this connection, a classification model is built from the sets of results data obtained from the free-viewing test and smooth pursuit and fixation tests.

Preferably, the modelling techniques comprise Gradient Boosted Decision Trees (GBDT) and Probabilistic Neural Networks (PNN) . Currently the PNN method is most able to describe the eye movement data and capable of providing reliable and accurate models. These methods are readily available, being in the public domain, and are implemented in public domain packages such as R, and in commercially-available packages such as DTREG.

In preferred embodiments, 9 median measures are taken for the free-viewing test, from (1) fixation number (frequency of fixations); (2) fixation duration (ms); (3) saccade number (frequency of saccades); (4) saccade duration (ms); (5) saccade amplitude (degrees); (6)saccade average velocity (deg/s); (7) saccade peak velocity (deg/s); (8) scanpath length (deg); (9) fixation dispersion (how clustered or dispersed the fixations made during the test).

Conveniently, the smooth pursuit test comprises the steps of: displaying a moving stimulus against a predetermined background in the middle of a display for a predetermined duration T; obtaining a set of eye movement measurements from the duration T excluding 1 second from the start and the last 1 second of a test; and estimating median values for Signal-to-Noise Ratio (SNR), Root Mean Square error (RMSE) and tracking gain (eye speed divided by stimulus speed). Conveniently, T is 20 seconds. If T > 20 seconds then the patient is likely to feel fatigue; if T < 20 seconds then calculation of the performance measures is prone to error.

The moving stimulus may be a red circle or a spot which changes colour (red, blue) at random intervals. The spot is 0.5 degree diameter.

The predetermined background can comprise a colour, black or a random backdrop of spots. When used, 100 spots are normally adequate to provide a textured backdrop. The position of the spot on the display is determined by random coordinates drawn from the Uniform Distribution.

Preferably, the stimulus moves in a horizontal sinusoid having a temporal frequency between 0.2 Hz to 0.6 Hz.

Conveniently, the stimulus can move in a 2D sinusoidal Lissajous pattern across the height and width of the screen having a temporal frequency between 0.2 Hz and 0.4 Hz.

In preferred embodiments, 10 median measures are taken for the smooth pursuit test, from (1) fixation number (frequency of fixations - interpreted as a period of continuous accurate pursuit or glissade); (2) fixation duration (ms); (3) saccade number (frequency of saccades); (4) saccade duration (ms); (5) saccade amplitude (degrees); (6)saccade average velocity (deg/s); (7) saccade peak velocity (deg/s); (8) signal-to-noise ratio (amount of accurate smooth pursuit compared with 'noisy' brief saccadic errors and adjustments); (9) root-mean-square-error (average positional error of the eye relative to the position of the target); (10) gain (ratio of overall speed of the eye during pursuit to the speed of the target).

In preferred embodiment where the stimulus moves in a 2D sinusoidal Lissajous pattern, 10 median measures are also taken for the smooth pursuit test for vertical eye movement behaviour.

The fixation stability test may comprise the steps of: displaying a central target, or a central target together with a flanking distracter for a predetermined duration T2; obtaining a set of fixation eye movement measurements; and calculating and obtaining median results from the fixation measurements separately for single and distracter conditions. Preferably, the predetermined duration T2 is 5 seconds.

In preferred embodiments, 5 median measures are taken for the fixation test, from (1) fixation number (frequency of fixations); (2) fixation duration (ms); (3) saccade number (frequency of saccades); (4) saccade amplitude (degrees); (5) scanpath length (deg).

The apparatus of the present invention is preferably configured to perform smooth pursuit and fixation tests, and combine measures consistent across these tests.

The apparatus of the present invention may be used in a system for identifying and classifying a psychiatric illnesses of a patient which system may comprise:-
using the apparatus in a method as described above to build an anonymous database of eye information data on individuals with psychiatric disorders and control subjects, using the apparatus in the method to test said patient and comparing the patient's test results against the database for identifying and classifying psychiatric illness.

Conveniently, the method or system is controlled by software running on a computer.

A computer program is preferably used for carrying out the above method or system, or a computer usable medium having control logic stored therein for causing a computer to carry out the above system or method.

An example of the present invention will now be described with reference to the accompanying drawings, of which:-
Figure 1 is a schematic drawing showing an embodiment of apparatus in accordance with the present invention in use; and,
Figure 2 is a flow diagram of a method performed using the embodiment of the apparatus in accordance with the present invention.

The apparatus 1 for psychiatric evaluation comprises a control means 2 and a display means 12 where the control means 2 is also connected to an accessible memory means 4 which holds a store of pre-selected images (the stimuli of a test). These images are carefully chosen generic pictures with a consideration of the cultural influences, so that they are non-evocative and neutral to the participants. They may for example be landscapes, people, faces, or objects. They are not moving images, but simply images of neutral emotion. Furthermore, based on these images a free-viewing test 102, where the participants need to look at the image, is prepared.

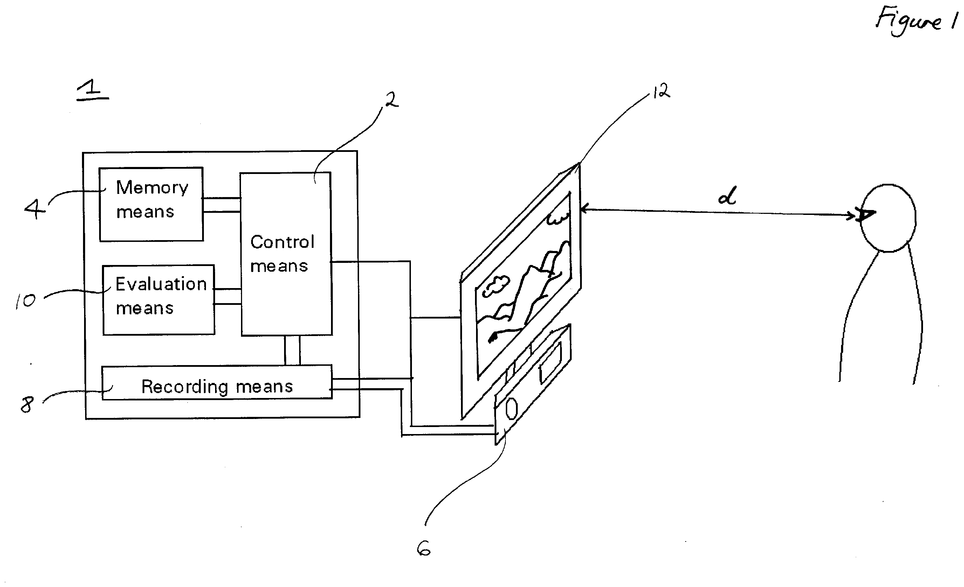

As shown in Figure 1, the control means 2 is also connected to the display means 12 so that the images can be displayed on the display means 12 according to the set up of the test. These images are displayed in a predetermined order and for a predetermined period of time.

The display means 12 may be a 19-inch (48cm) computer display (either as a 19-inch TFT display, or 21-inch CRT device with visible image scaled to match). There is no limitation to the size or the type of display units to be used, however, it is important that the presentation of the stimuli must be standardised.

For example, as illustrated in Figure 1, during the test the display means 12 is viewed from 70cm (distance (d) from screen to observer's eye) which can be measured by using a tape measure or if in a fixed environment measured from a head rest. The viewing distance (d, measured in degrees of visual angle) and the size of image/stimuli is kept constant so that the stimulus may subtend a constant visual area (28.1 h x 21.4v) thereby stimulating a standardised amount of visual cortex in the brain. This can be appreciated in terms of the number of eye movements required to inspect a 30cm square photograph viewed from 5 meters (i.e. virtually none) compared with inspection of the same photograph viewed from 0.5 meters (i.e. many).

Using the control means 2 and the memory means 4, the pre-selected images are automatically scaled to the native resolution of the display means 12. There may be discrepancy in the appearance of the images as the resolution of the images may vary depending on the size and the quality of the original image, however as long as the viewing distance (d) of the test is kept this will not affect the test.

In a modified free viewing part of the test, the order of presentation of the stimuli type is fixed, after choosing a predetermined number of images from the memory means 4. A total of 42 images may be used for each test run. This number has proved effective.

Each of the selected images is presented on the display means 12 for 5 to 10 seconds. A duration of 8 seconds has proven most effective to capture sufficient eye movements of the participants. When setting up the test this duration must be kept consistent throughout the free-viewing test for each of its runs. Practice trials are not used for free-viewing tests. However, calibration procedures are allowed.

During the test period, the control means 2 which is further connected to eye tracking means 6 and recording means 8, utilises these components to track and to record the eye movement of the test participants. Raw eye position data may be acquired between 120Hz and 1000Hz. A data rate at 250Hz or 500Hz has proven effective in collecting necessary data from the test. Once selected, the rate must be also kept consistent throughout the tests. Higher rates such as 1000 or 2000Hz may not afford any benefit, as the stimuli/task do not require very high resolution data. However, rates below 120Hz are not advisable because this may not sample eye position often enough to capture the fine details of eye movements induced by the stimuli.

The apparatus 1 may involve a helmet system with no head rest or a system where a chin and head rest are provided. This may depend on whether the participants can keep still during eye movement recording. The display means 12 may vary depending on which set-up is being used.

The apparatus 1 further comprises a plurality of similar themed stimuli/images which may be stored in the memory means 4, and the control means 2 takes images in turn in a predetermined sequence. As discussed, the images are provided on the display means 12 wherein themes of these images include landscapes, faces and common everyday objects. The images are intended to be neutral and the display order preferably remains consistent.

From the free-viewing test, 9 median measures can be taken: (1) fixation number (frequency of fixations); (2) fixation duration (ms); (3) saccade number (frequency of saccades); (4) saccade duration (ms); (5) saccade amplitude (degrees); (6)saccade average velocity (deg/s); (7) saccade peak velocity (deg/s); (8) scanpath length (deg); (9) fixation dispersion (how clustered or dispersed the fixations made during the test).

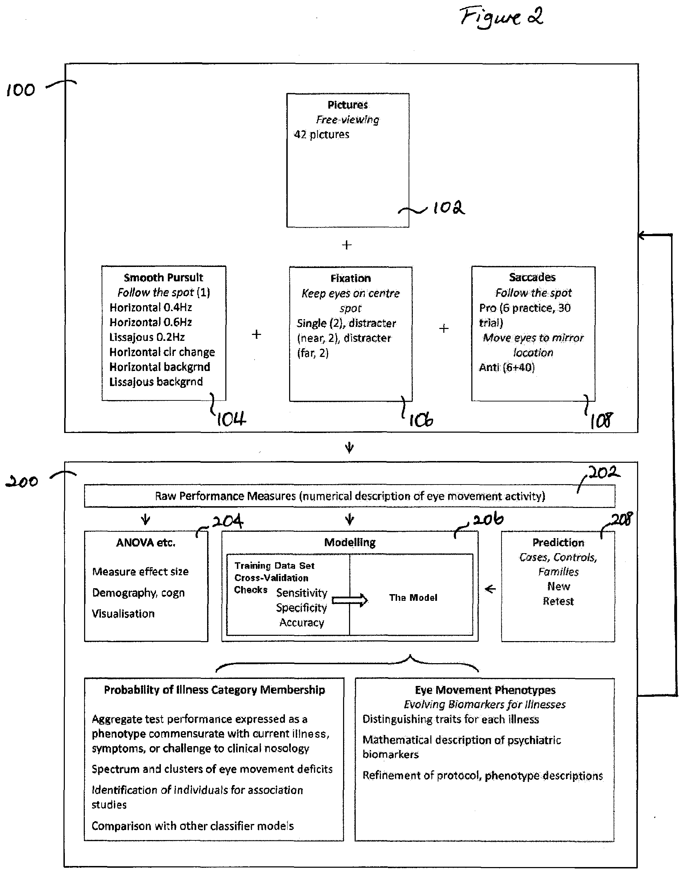

Figure 2 shows the control means 2 controlling the apparatus to perform one or more of smooth pursuit tests 104, fixation test 106, and saccade tests 108 along with the free-viewing test 102. In the apparatus of the present invention as claimed, the control means is configured to perform the smooth pursuit test 104 and the fixation test 106.

In the smooth pursuit test 104, the participant is required to follow a target as smoothly as possible with their eyes, once it starts moving. The target stimulus may be a solid red circle shown against a black background. The target stimulus may be a red spot or a spot which changes its colour into such as red or blue at random intervals. Viewed from 70cm the target is 0.5 deg in diameter.

The stimulus preferably moves in a horizontal sinusoid in the middle of the display, and in a 2D sinusoidal Lissajous pattern across the height and width of the screen.

At the experimenter's discretion participants are encouraged to undertake each of:
1. Horizontal 0.4Hz
2. Horizontal 0.6Hz
3. Lissajous 0.2Hz
4. Horizontal 0.4Hz changing (red/blue) target
5. Horizontal 0.4Hz against random 100-spot background
6. Lissajous 0.2Hz and 0.4Hz against random 100-spot background

Each trial lasts 20 seconds, however, the first and last 1 second of the trial are excluded in analysis so that the analysis is not affected by any pursuit initiation error, fatigue or anticipation of end of trial. There are no practice trials. Unlike other known smooth pursuit tests, no distinction is made amongst different types of saccades such as overshoots, catchup and backup. Only a tally is kept of the saccade events that have been observed during the smooth pursuits test 104. Furthermore, when the test results in poor pursuit data, the parameter estimation using an alternative epoch is performed at the evaluation stage 200.

From the smooth pursuit test, 10 median measures are taken: (1) fixation number (frequency of fixations - interpreted as a period of continuous accurate pursuit or glissade); (2) fixation duration (ms); (3) saccade number (frequency of saccades); (4) saccade duration (ms); (5) saccade amplitude (degrees); (6)saccade average velocity (deg/s); (7) saccade peak velocity (deg/s); (8) signal-to-noise ratio (amount of accurate smooth pursuit compared with 'noisy' brief saccadic errors and adjustments); (9) root-mean-square-error (average positional error of the eye relative to the position of the target); (10) gain (ratio of overall speed of the eye during pursuit to the speed of the target).

In the case the smooth pursuit test involves pursuing a 2D sinusoidal Lissajous pattern across the height and width of the screen, then 10 additional median measures of the above categories are taken for vertical eye movement behaviour.

The fixation test 106 requires the participant to maintain steady gaze only on a central target and to ignore flanking distracter targets when they are present. The central target may be 0.5 deg circular target. These distracter targets appear either to the left or right of the central target. The position of the distracter targets may be off to 1.43 or 2.86 deg on either side of the central target. Each condition is repeated twice. The target may be black on a mid-grey background.

The order of the test is fixed to:
1. Single
2. Distracter present close left
3. Distracter present close right
4. Distracter present far left
5. Distracter present far right
6. Single

These stimuli are pre-rendered as images and presented in exactly the same way as images in the free-viewing test 102. The fixed images are stored in the memory means 4. Each trial lasts 5 seconds and there are no practice trials.

From the fixation test, 5 median measures are taken: (1) fixation number (frequency of fixations); (2) fixation duration (ms); (3) saccade number (frequency of saccades); (4) saccade amplitude (degrees); (5) scanpath length (deg) .

The test presentation depends on the type of the participant being assessed. For example, the performance on the gap step and overlap conditions in dyslexia normally requires about 30 trials of each, in a pre-determined random order). With the psychiatric patients, for the same performance about 20 trials of each are required. There are equal numbers of left and right displacements and equal numbers of displacements at medium (300 pixels) and far (400 pixels) distances. The test lasts 4 seconds on average and there are 8 practice trials. A normalised saccade displacements data is collected so that eye movement performance is independent of stimulus properties. This also allows to obtain Signal to Noise Ratio (SNR) and gain which have been obtained in the smooth pursuit test 104.

In all tests described above, the lower fixation duration threshold is 80 ms whereas the upper threshold is unbounded. Furthermore, it is noted that any alteration to the test durations may have an adverse effect on the success of the modeling. Therefore consistency throughout the tests and collection of the data for the analysis is required.

In addition, the preference is to analyse data from the volunteer's dominant eye (which could be assessed using tests such as the 'hole-in-the-card' or keyhole test). Whenever possible binocular data can be obtained as a backup. However, the analysis may compensate for monocular recordings regardless of the dominance of the eye used due to problematic setup or calibration of the eye tracker.

As shown in Figure 2, once all tests 100 have been carried out, the sets of results data 202 from each of the test is passed onto the evaluation stage 200. The evaluation is carried out by building classification models 206 using supervised machine learning techniques. In addition, traditional methods such as analysis of variance 204 can also be used. Examples of such learning techniques are Gradient Boosted Decision Trees (GBDT) and Probabilistic Neural Networks (PNN) methods. Obtaining large data sets aids in tweaking the parameters of the analyses to be less critical. The parameters such as variable weighting can be adjusted depending on the purpose of the tests.

Each build of the model to incorporate new patient groups will alter the importance of individual tests and test measures.

In this connection, the control means attributes an importance weighting to the results data set from the modified free viewing test, and the results data set from said smooth pursuit and fixation tests.

Examples of how the models are built are illustrated with reference to the following models 1, 2, 3 and 3a. As shown, each build of the model to incorporate new patient groups will alter the importance of the individual tests and test measure. The relative importance of measures is given for a number of builds. The models shown in tables 1, 2 and 3 use 5 tests (free-viewing, fixation stability, and 3 pursuit tasks).

Model 1 relates not to the invention, but to controls and Schizophrenia. Classification performance for training and leave-one-out cross validation procedures are shown below. Average sensitivity, specificity and accuracy values are given in italics. Cross-validation was used to examine how well the model might generalise when presented with new data. More aggressive methods of cross validation such as 10% hold back can be used. However, there is a risk that important information (participant variance) would be lost as much as weaknesses in the model might be highlighted, when it is applied to the modest sample sizes used in this model. Nevertheless, these steps are necessary to limit the risk of the model being over-fitted to noisy data. Training set performance alone does not adequately reflect the model's efficiency or success, and had there been a perfect classification under cross validation then it is likely that there is a fault in the method. Schizophrenia cases include data from patients we tested in Germany (n=27). The top-10 most influential measures in determining model structure are also shown in a table.
con = undiagnosed controls, scz = schizophrenia, Sens = sensitivity, Spec = specificity, Acc = accuracy

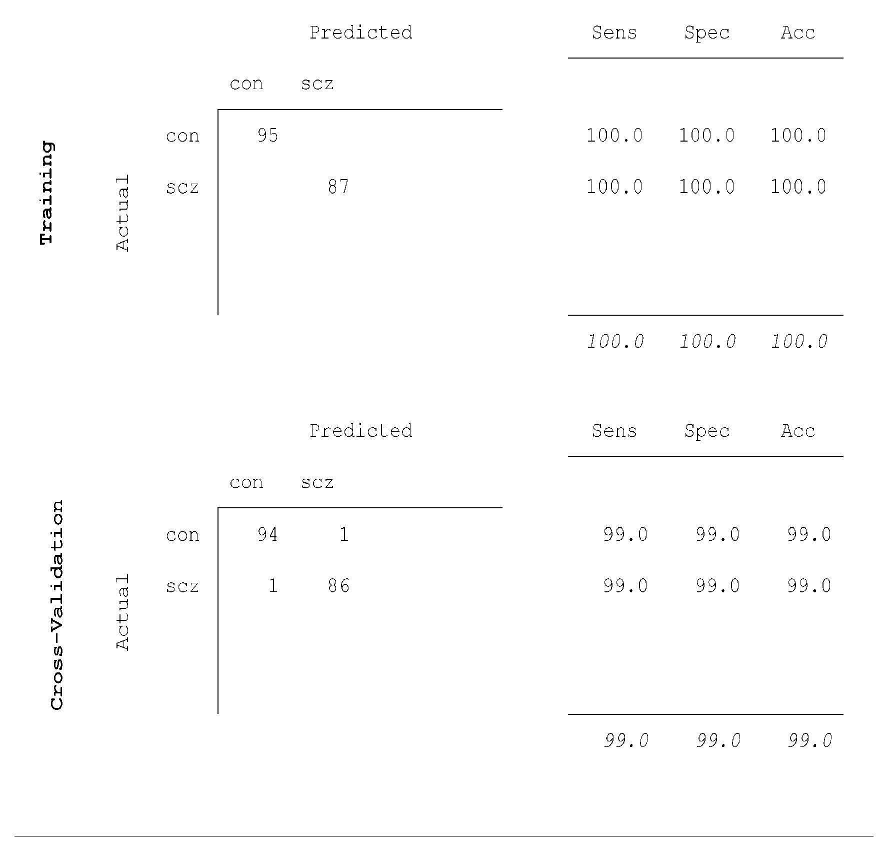

| **Test** | **Subtest** | **Measure** | **Importance** |
|---|---|---|---|
| Free-viewing | | Fixation dispersion | 100.0 |
| Fixation | Distracter | Scanpath length | 33.2 |
| Pursuit | Lissajous .2Hz | Saccade amplitude | 23.2 |
| Pursuit | Horizontal .4Hz | Saccade peak velocity | 16.7 |
| Free-viewing | | Fixation duration | 16.3 |
| Pursuit | Lissajous .4Hz | Gain (vertical) | 15.1 |
| Pursuit | Lissajous .2Hz | Gain (horizontal) | 14.0 |
| Free-viewing | | Scanpath length | 7.0 |
| Pursuit | Lissajous .2Hz | Fixation duration | 6.7 |
| Pursuit | Lissajous .2Hz | Saccade average velocity | 6.5 |

Model 2 relates to controls, Schizophrenia and Bipolar Disorder. The sophistication of the network modelling approach allows bipolar cases to be distinguished from schizophrenia with high accuracy. These results suggest that there may be unique eye movement phenotypes in these illnesses.
con = undiagnosed controls, scz = schizophrenia, bpad = bipolar affective disorder, Sens = sensitivity, Spec = specificity, Acc = accuracy

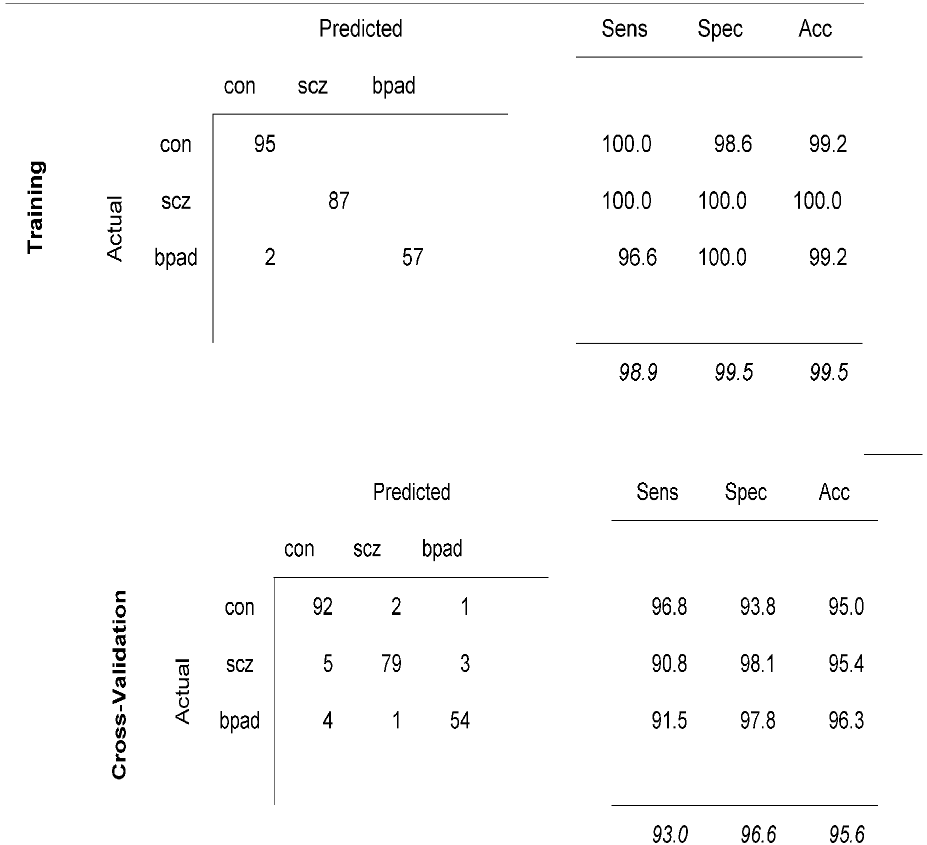

| **Test** | **Subtest** | **Measure** | **Importance** |
|---|---|---|---|
| Pursuit | Horizontal .4Hz | Gain | 100.0 |
| Pursuit | Lissajous .2Hz | Gain (horizontal) | 96.5 |
| Pursuit | Lissajous .4Hz | Gain (vertical) | 87.0 |
| Free-viewing | | Fixation dispersion | 85.0 |
| Pursuit | Lissajous .2Hz | Gain (vertical) | 35.9 |
| Fixation | Distracter | Scanpath length | 19.2 |
| Pursuit | Horizontal .4Hz | Saccade peak velocity | 18.9 |
| Pursuit | Lissajous .2Hz | Saccade amplitude | 15.4 |
| Free-viewing | | Scanpath length | 11.5 |
| Fixation | Distracter | Saccade frequency | 8.2 |

Model 3 relates to controls, Schizophrenia, Bipolar Disorder and Major Depressive Disorder. Data from 20 depressed cases (tabulated at the time of this report) are distinguished with surprising accuracy, with subtle consequences for the other categories. We want to recruit further MDD cases to improve the balance of sample numbers. Schizoaffective cases have not been included because supervised classification cannot learn enough about this group from a small sample size.
con = undiagnosed controls, scz = schizophrenia, bpad = bipolar affective disorder, mdd = major depressive disorder, Sens = sensitivity, Spec = specificity, Acc = accuracy

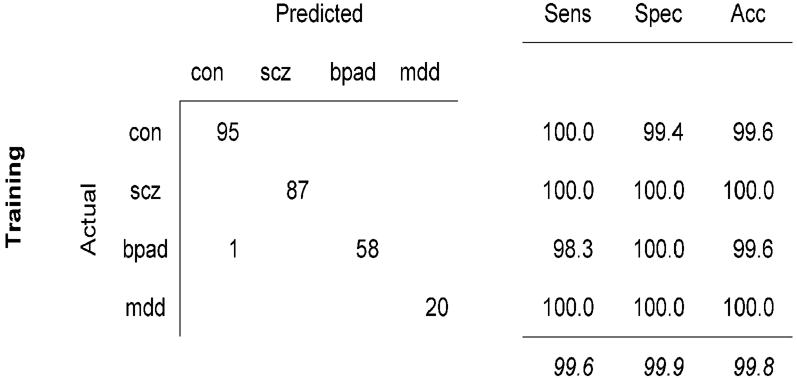

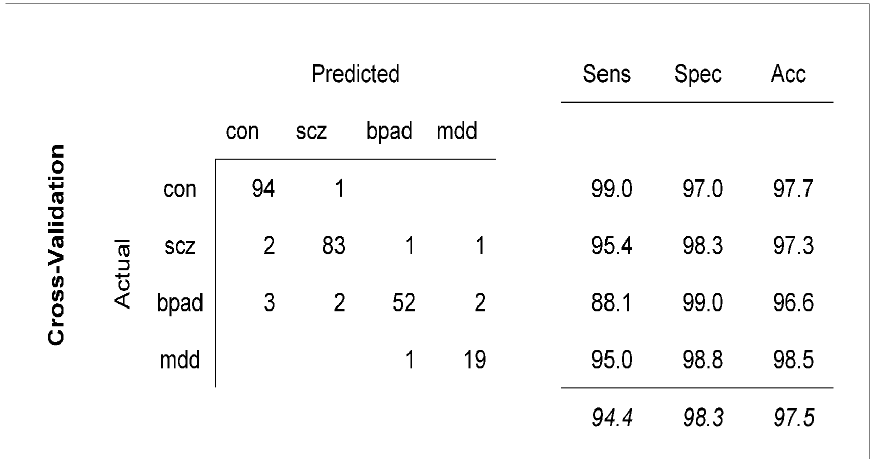

| **Test** | **Subtest** | **Measure** | **Importance** |
|---|---|---|---|
| Free-viewing | | Fixation dispersion | 100.0 |
| Pursuit | Lissajous .2Hz | Gain (horizontal) | 66.9 |
| Pursuit | Lissajous .2Hz | Gain (vertical) | 58.0 |
| Pursuit | Horizontal .4Hz | Saccade duration | 41.9 |
| Pursuit | Lissajous .4Hz | Saccade frequency | 33.2 |
| Free-viewing | | Saccade average velocity | 31.4 |
| Fixation | Distracter | Scanpath length | 31.3 |
| Free-viewing | | Saccade peak velocity | 27.7 |
| Pursuit | Horizontal .4Hz | Saccade peak velocity | 27.6 |
| Pursuit | Horizontal .4Hz | Gain (horizontal) | 25.8 |

Model 3 can be modified to use only 3 tests (picture viewing, Lissajous 0.2Hz pursuit, fixation stability) with a view to refining the testing procedure. Model 3a shows such simplified network model relating to controls, Schizophrenia, Bipolar Disorder and Major Depressive Disorder. Even in such case, separation of cases and controls remains impressive using performance measures from just three tests.
con = undiagnosed controls, scz = schizophrenia, bpad = bipolar affective disorder, mdd = major depressive disorder, Sens = sensitivity, Spec = specificity, Acc = accuracy

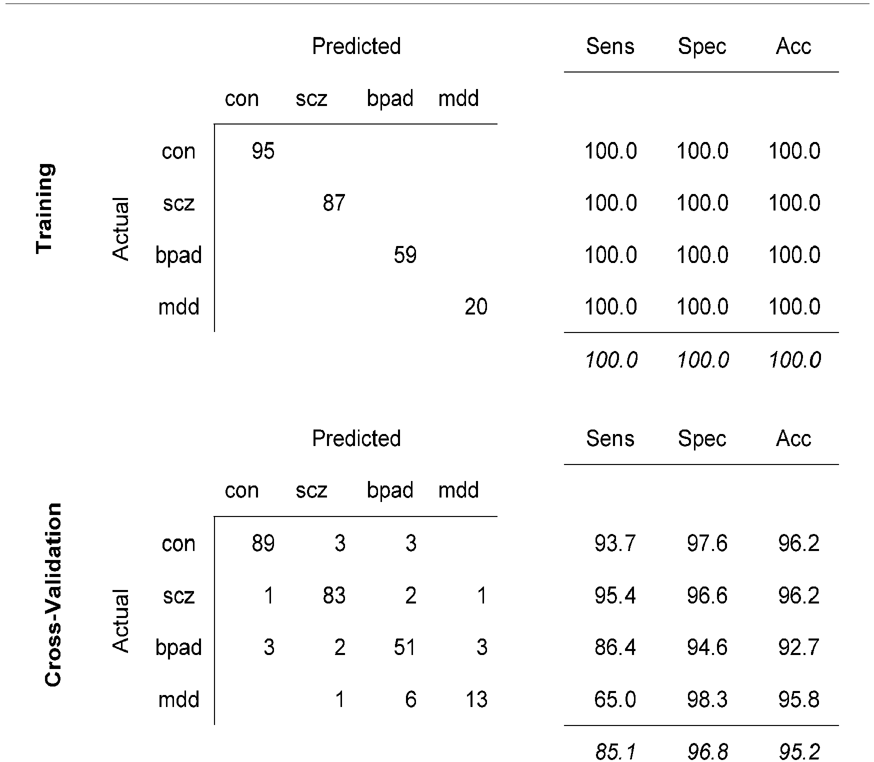

| **Test** | **Subtest** | **Measure** | **Importance** |
|---|---|---|---|
| Free-viewing | | Fixation dispersion | 100.0 |
| Pursuit | Lissajous .2Hz | Gain (vertical) | 71.6 |
| Pursuit | Lissajous .2Hz | Gain (vertical) | 37.3 |
| Fixation | Distracter | Fixation duration | 34.5 |
| Free-viewing | | Saccade amplitude | 33.8 |
| Free-viewing | | Saccade average velocity | 26.7 |
| Fixation | Distracter | Scanpath length | 20.9 |
| Fixation | Distracter | Saccade amplitude | 16.7 |
| Free-viewing | | Scanpath length | 14.4 |
| Fixation | Single | Scanpath length | 10.8 |

Models 3 and 3a show that, even if the number of tests has changed the importance of free-viewing (fixation dispersion) test remains 100.0 whereas the importance of Lissajous 0.2Hz pursuit and fixation stability has been adjusted. Furthermore, There can be 55 different measures, which can be obtained by going through all the tests to build a model. However, amongst these 55 measures free-viewing fixation dispersion has highest influence values with the median 96.96 with the range of 66 to 109.

In the preferred embodiments, 10 measures are hence chosen for use in the formulation of the models. Of these, the free viewing (fixation dispersion) measure has a consistently high importance weighting, with the remaining measures generally contributing to diminishing degrees in an algorithm for determining the evaluation model.

Once the classification models are established, they are refined and used to predict an illness of an individual 208. In this connection, the models are refined when new tests or retests are carried out on patients or control subjects. The patients and the control subjects who have been tested may be retested to check whether there have been any changes to their performance.

In the case where an individual cannot complete a test, this leaves missing data before modeling 206 can take place. In such a case, the traditional method replaces missing values with the mean or median of the relevant group's values for that variable. However, according to present invention, surrogate splitters (GBDT) or surrogate variables (PNN) replace missing data at the evaluation stage 200. Surrogate values are interpolated from the individual's available data based on their group's characteristics. This allows incomplete test results data which are a feature of clinical experiments to be reflected in the analysis.

Each of the tests and the modeling can be customised depending on the individual participants, illnesses etc. In the example given, the apparatus is applied to schizophrenia. However, in this regard, the same may be applicable for delineating other psychiatric phenotypes such as bipolar affective disorder, and major depressive disorders.

The machine learning model using data from a limited number of psychiatric cases with various diagnoses together with normal control subjects shows remarkable predictive ability to correctly classify new cases and control subjects. Therefore, the memory means 4 can be designed to contain data from a large sample sizes with defined clinical psychiatric disorders as well as individuals with ill defined conditions, high risk family members and individuals with dyslexia etc. Furthermore, the memory means 4 can also be constantly added to and refined.

The information about the variation in patients and control subjects can be exploited by refining performance measurement and analysing these measures using sophisticated algorithms. This indicates an accurate trait or biomarker of risk of psychiatric illnesses using an eye movement phenotype is possible. The profile of the abnormalities detected may be able to stratify psychiatric illnesses into clinically relevant subcategories. With this in mind, the apparatus is designed in such a way that it can be used in a clinical setting with minimal training and be as compact as a personal laptop or desktop computer.

In this connection, the apparatus may be used for the clinical assessment, diagnosis and treatment of individuals with schizophrenia, bipolar affective disorder and major depressive disorders. Relevant treatment can also include drug trials by the pharmaceutical industry.

The apparatus may also be used in the evaluation of individuals at high risk of said psychiatric disorders who may be only at the time of assessment be demonstrating vague psychiatric symptoms. However if the presence of specific patterns of eye movement abnormalities put the individual into the ultra high risk category clinical intervention would probably be indicated. Furthermore, the apparatus provides an anonymised database of eye movement information on large numbers of individuals with said disorders and control subjects with which new clinical assessments and screening tests etc can be compared.

In addition there are certain occupations where potential recruits are carefully screened for the absence of psychiatric disorders. These include the armed forces, airline pilots, offshore workers on oil rigs etc. The above described eye movement tests can be an invaluable adjunct to clinical history and examination. For example, with regard to the armed forces, they could be used not only to screen out individuals at high risk of said psychiatric disorders but also used as baseline measurements among recruits themselves. This is possible because the tests are normally stable over time so that if there are any subsequent changes in baseline measurements this may provide biological evidence of traumatic brain injury (TBI) or post-traumatic stress disorder (PTSD).

As described above, any eye movement tests are likely to be used in combination with clinical evaluation and genotypic information.

It will be understood that the embodiment illustrated above shows an application of the invention only for the purposes of illustration. In practice the invention may be applied to many different configurations, the detailed embodiments being straightforward for those skilled in the art to implement.

In this respect, off-the shelf hardware such as EyeLink I (provides a helmet system and no head rest) can be used for building the apparatus and EyeLink 2000 eye tracker (provides a chin/head rest) which consists of a 'host PC' which acquires eye gaze data communicating with a 'subject PC' which presents the images can also be used.

## Claims

1. Apparatus (1) for use in a method for delineating schizophrenia, bipolar disorder, and optionally major depressive disorders, comprising:
memory means (4) for holding a store of pre-selected images;
display means (12) for displaying a succession of said images to a patient as a free-viewing test (102);
eye tracking means (6) for tracking eye movement of the patient;
recording means (8) for recording eye movement data corresponding to a patient's eye movement in response to the display of said succession of images;
control means (2) for controlling the display means to display the images to the patient in a predetermined order and for a predetermined period of time, and for controlling the recording means to record said eye movement data and displayed image data over said period of time; and
evaluation means (10) for evaluating said eye movement data and displayed image data to produce a first results data set;
wherein the control means is further configured to control the apparatus to perform a smooth pursuit test (104), and a fixation test (106), and to take the results data sets from said tests to refine the first results data set in identifying a psychiatric profile of the patient;
wherein the apparatus is further configured to:
carry out an evaluation stage by building classification models using supervised machine learning techniques based on the sets of test results data;
and to use the classification models to predict schizophrenia, bipolar affective disorder, and optionally major depressive disorders of an individual.

2. Apparatus as claimed in claim 1, further comprising a memory means for storing a plurality of neutral images, and said control means provides that each of said images from said memory means are displayed in turn in a predetermined sequence.

3. Apparatus as claimed in claim 1, wherein the control means attributes an importance weighting to measures from the first results data set, and the second results data set from said smooth pursuit tests and fixation test.

4. Apparatus as claimed in any preceding claim, wherein 10 measures are used from the collected eye movement data for building an evaluation model.

5. Apparatus as claimed in claim 4, wherein said measures include a free viewing measure.

6. Apparatus as claimed in any preceding claim, wherein 9 median measures are taken for the free-viewing test, from (1) fixation number, i.e. frequency of fixations; (2) fixation duration; (3) saccade number, i.e. frequency of saccades; (4) saccade duration; (5) saccade amplitude; (6)saccade average velocity; (7) saccade peak velocity; (8) scanpath length; (9) fixation dispersion, i.e. how clustered or dispersed the fixations made during the test.

7. Apparatus as claimed in any preceding claim, wherein the smooth pursuit test comprises the steps of:
displaying a moving stimulus against a predetermined background in the middle of a display for a predetermined duration;
obtaining a set of eye movement measurements from the duration excluding 1 second from the start and the last 1 second of a test; and
estimating median values for Signal-to-Noise Ratio, Root Mean Square error and tracking gain.

8. Apparatus as claimed in claim 7, wherein 10 median measures are taken for the smooth pursuit test, from (1) fixation number, i.e. frequency of fixations - interpreted as a period of continuous accurate pursuit or glissade; (2) fixation duration; (3) saccade number, i.e. frequency of saccades; (4) saccade duration; (5) saccade amplitude; (6)saccade average velocity; (7) saccade peak velocity; (8) signal-to-noise ratio, i.e. amount of accurate smooth pursuit compared with "noisy" brief saccadic errors and adjustments; (9) root-mean-square-error, i.e. average positional error of the eye relative to the position of the target; (10) gain, i.e. ratio of overall speed of the eye during pursuit to the speed of the target.

9. Apparatus as claimed in claim 8, wherein the 10 median measures are taken for the smooth pursuit test for vertical eye movement behaviour.

10. Apparatus as claimed in any preceding claim, wherein the fixation stability test comprises the steps of:
displaying a central target, or a central target and a flanking distracter for a predetermined duration;
obtaining a set of fixation eye movement measurements; and
calculating and obtaining median results from the fixation measurements separately for single and distracter conditions.

11. Apparatus as claimed in claim 10, wherein 5 median measures are taken for the fixation test, from (1) fixation number, i.e. frequency of fixations; (2) fixation duration; (3) saccade number, i.e,. frequency of saccades; (4) saccade amplitude; (5) scanpath length.

## Patentansprüche

1. Apparat (1) für die Nutzung in einem Verfahren für die Beschreibung von Schizophrenie, bipolarer Störung und optional größeren depressiven Störungen, umfassend:
Speichergerät (4) für das Speichern von vorausgewählten Bildern;
Anzeigegerät (12) für das Anzeigen einer Abfolge dieser Bilder an einen Patienten als freien Sehtest (102);
Eye-Tracking-Gerät (6) für die Nachverfolgung einer Augenbewegung des Patienten;
Aufzeichnungsgerät (8) für das Aufzeichnen der Augenbewegungsdaten entsprechend der Augenbewegung eines Patienten infolge der Anzeige der Abfolge von Bildern;
Steuervorrichtung (2) für das Steuern des Anzeigegeräts, um dem Patienten die Bilder in einer vorbestimmten Reihenfolge und für einen vorbestimmten Zeitraum zu zeigen, und für das Steuern des Aufzeichnungsgeräts, um diese Augenbewegungsdaten und die angezeigten Bilddaten über diesen Zeitraum aufzuzeichnen; und
Bewertungsvorrichtung (10) für das Bewerten dieser Augenbewegungsdaten und der angezeigten Bilddaten, um einen ersten Ergebnisdatensatz zu erzeugen;
wobei die Steuervorrichtung ferner konfiguriert ist, den Apparat zu steuern, um einen Smooth Pursuit Test (104) und einen Fixationstest (106) auszuführen, und die Ergebnisdatensätze dieser Tests zu nehmen, um die ersten Ergebnisdatensätze durch Identifizieren eines psychiatrischen Profils des Patienten zu verfeinern;
wobei der Apparat ferner für Folgendes konfiguriert ist:
Ausführen einer Bewertungsphase durch Erstellen von Klassifizierungsmodellen unter Verwendung von überwachten Maschinenlerntechniken, basierend auf den Testergebnisdatensätzen,
und Verwenden der Klassifizierungsmodelle, um Schizophrenie, bipolare affektive Störung und optional größere depressive Störungen eines Individuums vorherzusagen.

2. Apparat nach Anspruch 1, ferner umfassend ein Speichergerät für das Speichern einer Vielzahl von neutralen Bildern, die Steuervorrichtung sorgt dafür, dass jedes dieser Bilder aus diesem Speichergerät dann in einer vorbestimmten Abfolge angezeigt wird.

3. Apparat nach Anspruch 1, wobei die Steuervorrichtung den Maßnahmen aus dem ersten Ergebnisdatensatz und aus dem zweiten Ergebnisdatensatz der Smooth Pursuit Tests und dem Fixationstest eine Bedeutungsgewichtung zuteilt.

4. Apparat nach einem der vorhergehenden Ansprüche, wobei 10 Messungen aus den gesammelten Augenbewegungsdaten für das Erstellen eines Bewertungsmodells verwendet werden.

5. Apparat nach Anspruch 4, wobei diese Messungen eine freie Sehtest-Messung beinhalten.

6. Apparat nach einem der vorhergehenden Ansprüche, wobei 9 Median-Messungen für den freien Sehtest herangezogen werden, von (1) Fixationszahl, d.h. Frequenz der Fixationen; (2) Fixationsdauer; (3) Sakkadenzahl, d.h. Frequenz der Sakkaden; (4) Sakkadendauer (5) Sakkadenhöhe; (6) Sakkaden-Durchschnittsgeschwindigkeit; (7) Sakkaden-Spitzengeschwindigkeit; (8) Scanpath-Länge; (9) Fixationsverteilung, d.h. wie geclustert oder verteilt die bei dem Test auftretenden Fixationen sind.

7. Apparat nach einem der vorhergehenden Ansprüche, wobei der Smooth Pursuit Test die folgenden Schritte umfasst:
Anzeigen eines Bewegungsstimulus gegen einen vorbestimmten Hintergrund in der Mitte einer Anzeige für eine vorbestimmte Dauer;
Erlangen eines Satzes von Augenbewegungsmessungen während der Dauer, wobei 1 Sekunde vom Start und die letzte Sekunde eines Tests ausgeschlossen werden; und
Schätzen von Medianen für das Signal-Rausch-Verhältnis, Fehler im quadratischen Mittelwert und erneutes Nachverfolgen.

8. Apparat nach Anspruch 7, wobei 10 Median-Messungen für den Smooth Pursuit Test vorgenommen werden, von (1) Fixationszahl, d.h. Frequenz der Fixationen - interpretiert als Zeitspanne des kontinuierlichen akkuraten Pursuit oder Glissade; (2) Fixationsdauer; (3) Sakkadenzahl, d.h. Frequenz der Sakkaden; (4) Sakkadendauer; (5) Sakkadenhöhe; (6) Sakkaden-Durchschnittsgeschwindigkeit; (7) Sakkaden-Spitzengeschwindigkeit; (8) Signal-Rausch-Verhältnis, d.h. Betrag des akkuraten Smooth Pursuit im Vergleich zu "lauten", kurzen sakkadischen Fehlern und Anpassungen; (9) Fehler im quadratischen Mittelwert, d.h. durchschnittliche Positionsfehler des Auges im Verhältnis zur Position des Ziels; (10) Verstärkungsgrad, d.h. das Verhältnis der Gesamtgeschwindigkeit des Auges während des Pursuits zur Geschwindigkeit des Ziels.

9. Apparat nach Anspruch 8, wobei die 10 Median-Messungen für den Smooth Pursuit Test für vertikales Augen-Bewegungs-Verhalten genommen werden.

10. Apparat nach einem der vorhergehenden Ansprüche, wobei der Fixations-Stabilitäts-Test die folgenden Schritte umfasst:
Anzeige eines zentralen Ziels oder eines zentralen Ziels und eines flankierenden Distraktors für eine vorbestimmte Dauer;
Erlangen eines Satzes von Fixations-Augenbewegungs-Messungen;
und
Berechnen und Erlangen von Median-Ergebnissen aus den Fixationsmessungen separat für Einzel- und Distraktorbedingungen.

11. Apparat nach Anspruch 10, wobei 5 Median-Messungen für den Fixationstest vorgenommen werden, aus (1) Fixationszahl, d.h. Frequenz der Fixationen; (2) Fixationsdauer; (3) Sakkadenzahl, d.h. Frequenz der Sakkaden; (4) Sakkadenhöhe; (5) Scanpath-Länge.

## Revendications

1. Appareil (1) destiné à être utilisé dans une méthode servant à la délimitation de la schizophrénie, du trouble bipolaire et, éventuellement, des troubles dépressifs majeurs, comprenant :
un moyen de mémorisation (4) destiné à stocker une mémoire d'images présélectionnées ;
un moyen d'affichage (12) destiné à afficher une succession desdites images à un patient sous la forme d'un test de vision libre (102) ;
un moyen de suivi des yeux (6) destiné à suivre le mouvement des yeux du patient ;
un moyen d'enregistrement (8) destiné à enregistrer les données de mouvement des yeux correspondant au mouvement des yeux d'un patient en réponse à l'affichage de ladite succession d'images ;
un moyen de commande (2) destiné à commander le moyen d'affichage afin d'afficher les images au patient dans un ordre prédéfini et pendant une période de temps prédéfinie, et destiné à commander le moyen d'enregistrement afin d'enregistrer les données relatives au mouvement des yeux et les données relatives à l'image affichée pendant ladite période de temps ; et
un moyen d'évaluation (10) destiné à évaluer lesdites données relatives au mouvement des yeux et les données relatives à l'image affichée afin de produire un premier ensemble de données de résultats ;
dans lequel, le moyen de commande est en outre configuré pour commander l'appareil afin qu'il effectue un test de poursuite en douceur (104) et un test de fixation (106) et pour prendre les ensembles de données de résultats à partir desdits tests afin d'affiner le premier ensemble de données de résultats dans l'identification d'un profil psychiatrique du patient ;
dans lequel l'appareil est configuré en outre pour :
réaliser une étape d'évaluation en construisant des modèles de classification à l'aide de techniques d'apprentissage automatique supervisé basées sur les ensembles de données de résultats des tests ;
et utiliser les modèles de classification pour prédire la schizophrénie, les troubles affectifs bipolaires et, éventuellement, les troubles dépressifs majeurs d'un individu.

2. Appareil selon la revendication 1, comprenant en outre un moyen de mémorisation destiné à mémoriser une pluralité d'images neutres et ledit moyen de commande assure que chacune des images provenant dudit moyen de mémorisation est affichée à tour de rôle dans une séquence prédéfinie.

3. Appareil selon la revendication 1, dans lequel le moyen de commande attribue une pondération d'importance aux mesures provenant du premier ensemble de données de résultats et du second ensemble de données de résultats provenant desdits tests de poursuite en douceur et test de fixation.

4. Appareil selon l'une des revendications précédentes, dans lequel 10 mesures sont utilisées à partir des données de mouvement des yeux destinées à construire un modèle d'évaluation.

5. Appareil selon la revendication 4, dans lequel lesdites mesures comprennent une mesure de visualisation libre.

6. Appareil selon l'une des revendications précédentes, dans lequel 9 mesures médianes sont prises pour le test de vision libre, parmi (1) le nombre de fixations, c.-à-d. la fréquence des fixations ; (2) la durée des fixations ; (3) le nombre de saccades, c.-à-d. la fréquence des saccades ; (4) la durée des saccades ; (5) l'amplitude des saccades ; (6) la rapidité moyenne des saccades ; (7) la rapidité maximale des saccades ; (8) la longueur de la trajectoire de balayage ; (9) la dispersion des fixations, c.-à-d. le degré de regroupement ou de dispersion des fixations effectuées pendant le test.

7. Appareil selon l'une des revendications précédentes, dans lequel le test de poursuite en douceur comprend les étapes suivantes :
l'affichage d'un stimulus en mouvement sur un fond prédéfini au milieu d'un dispositif d'affichage pendant une durée prédéfinie ;
l'obtention d'un ensemble de mesures du mouvement des yeux à partir de la durée excluant 1 seconde entre le début et la dernière 1ère seconde d'un test ; et
l'estimation des valeurs médianes pour le rapport signal/bruit, de l'erreur quadratique moyenne et du gain de suivi.

8. Appareil selon la revendication 7, dans lequel 10 mesures médianes sont prises pour le test de poursuite en douceur, à partir du (1) nombre de fixations, c.-à-d. la fréquence des fixations, interprétée comme une période de poursuite précise continue ou de glissade ; (2) la durée des fixations ; (3) le nombre de saccades, c.-à-d. la fréquence des saccades ; (4) la durée des saccades ; (5) l'amplitude des saccades ; (6) la rapidité moyenne des saccades ; (7) la rapidité maximale des saccades ; (8) le rapport signal/bruit, c.-à-d. la quantité de poursuites en douceur précise comparée aux erreurs et ajustements saccadiques brefs « bruyantes » ; (9) l'erreur quadratique moyenne, c.-à-d. l'erreur de position moyenne de l'œil par rapport à la position de la cible ; (10) le gain, c.-à-d. le rapport entre la vitesse globale de l'œil pendant la poursuite et la vitesse de la cible.

9. Appareil selon la revendication 8, dans lequel les 10 mesures médianes sont prises pour le test de poursuite en douceur destiné au comportement du mouvement vertical des yeux.

10. Appareil selon l'une des revendications précédentes, dans lequel le test de stabilité de la fixation comprend les étapes suivantes :
l'affichage d'une cible centrale, ou d'une cible centrale et d'un distracteur latéral pendant une durée prédéfinie ;
l'obtention d'un ensemble de mesures de fixation du mouvement des yeux ;
et
le calcul et l'obtention des résultats médians provenant des mesures de fixation séparément pour les conditions simples et les conditions de distraction.

11. Appareil selon la revendication 10, dans lequel 5 mesures médianes sont prises pour le test de fixation, parmi (1) le nombre de fixations, c.-à-d. la fréquence des fixations ; (2) la durée des fixations ; (3) le nombre de saccades, c.-à-d. la fréquence des saccades ; (4) l'amplitude des saccades ; (5) la longueur de la trajectoire de balayage.
